# EUROPÄISCHE PATENTANMELDUNG

(11) **EP 0 563 423 A1**
(43) Veröffentlichungstag der Anmeldung: **06.10.1993**
(21) Anmeldenummer: 92105552.1
(22) Anmeldetag: 31.03.1992
(51) Int. Cl.: A01H 4/00

(54) **Verfahren zur Adaptierung von durch Gewebekultur vermehrten Pflanzen für unmittelbares Auspflanzen ins Freiland**

(71) Anmelder: A. & F. FÖGLEIN KERTESZETI Bt., H-1028 Budapest (HU); Fa. A. VERSCHOOR, NL-2192 Hillegom (NL)
(72) Erfinder: Föglein, Ferenc, Dr., H-1028 Budapest (HU)
(74) Vertreter: von Füner, Alexander, Dr.

(57) **Zusammenfassung**

Verfahren zur Adaptierung von durch Gewebekultur vermehrten Pflanzen für unmittelbares Auspflanzen ins Freiland, für das kennzeichnend ist, daß man die in bekannter Weise durch Gewebekultur vermehrten Pflanzen
a) in einem oder in mehreren Schritten auf Nährböden weiterzüchtet, deren osmotischer Druck 0,2-0,4 M Saccharosegehalt entspricht, wobei im Falle einer mehrstufigen Anzucht der osmotische Druck des Nährbodens innerhalb des angegebenen Bereiches von Stufe zu Stufe auf einen höheren Wert eingestellt wird, und/oder
b) wenigstens 30 Tage lang bei 2-10 °C lagert, und die auf diese Weise erhaltenen Pflanzen unmittelbar ins Freiland auspflanzt oder für diesen Zweck vermarktet.

## Beschreibung

Die Erfindung betrifft ein Verfahren zur Adaptierung, Akklimatisierung von durch Gewebekultur vermehrten Pflanzen für unmittelbares Auspflanzen ins Freiland.

Die Techniken zur Vermehrung von Pflanzen durch Mikrovermehrung, Gewebekultur gewinnen weltweit an Bedeutung. Sie bestehen im Prinzip darin, daß unter sterilen Bedingungen aus verhältnismäßig kleinen Pflanzenteilen auf Hormone, Vitamine und Nährstoffe enthaltenden Nährböden komplette Pflanzen gezüchtet werden. Durch entsprechende Zusammensetzung der Nährböden kann die Bildung zahlreicher pflanzlicher Triebe beziehungsweise auch die Bewurzelung der entwickelten Triebe erzielt werden

Die auf diese Weise bewurzelten Pflanzen sind jedoch nicht geeignet, sofort in den Garten oder aufs Feld gepflanzt zu werden, denn ihre Gewebestruktur ist locker, sie sind empfindlich gegen UV-Strahlen, gegen das Austrocknen und gegen veränderliche Temperatur. Durch die Sonnenstrahlen werden sie verbrannt, sie verlieren ihren Wassergehalt und gehen schließlich zugrunde. Deswegen verfügen Gewebekulturlaboratorien gewöhnlich über Gewächshäuser, Folienzelte oder sonstige Objekte mit geschlossenem Luftraum, oder aber sie pachten wenigstens derartige sog. "Abhärtungs"flächen (E. F. George und P. D. Sherrington: Plant Propagation by Tissue Culture, Exegetics Ltd., S. 42, England, 1984).

In den Gewächshäusern und Folienzelten werden die Pflanzen allmählich an rauhere Bedingungen, an eine niedrigere relative Luftfeuchtigkeit, stärkere Temperaturschwankungen und an intensives Licht gewöhnt. Dieser Gewöhnungsprozeß nimmt etwa 6-8 Wochen in Anspruch, und dabei gehen etwa 10-80 % der ursprünglich im Laboratorium gezüchteten Pflanzen zugrunde. Dadurch geht nicht nur viel Arbeitsaufwand und Material verloren, sondern es muß auch berücksichtigt werden, daß die im isolierten Luftraum erfolgende Abhärtung beträchtliche Kosten verursacht. Das Folienzelt ist etwas billiger als das Gewächshaus, jedoch ist das Betreiben des ersteren im Winter und im zeitigen Frühjahr problematisch.

Es bestand demnach das Bedürfnis nach einem Gewebekulturverfahren, mit dem sofort unter freiem Himmel auspflanzbare Pflanzen herangezogen werden können. Ein derartiges Verfahren existiert bisher nicht, die durch Gewebekultur gezüchteten Pflanzen werden überall nur nach entsprechender Adaptierung ins Freie gepflanzt.

Die Erfindung geht davon aus, daß unter Freilandbedingungen die auf die Pflanzen einwirkenden Faktoren, wie zum Beispiel Temperaturschwankungen, zeitweiliger Wassermangel, Niederschlag, Wind usw., verantwortlich für die Entstehung der "abgehärteten" Pflanze, für diejenigen Veränderungen sind, die aus dem durch Gewebekultur gezüchteten empfindlichen, eine lockere Gewebestruktur aufweisenden Setzling die den Habitus ihrer Art annehmenden Pflanze ausbilden. Wenn die genannten Faktoren bereits im Zeitabschnitt der Gewebekultur zur Wirkung gebracht werden könnten, brauchte die auf sterilem Nährboden bewurzelte Pflanze nicht einem zeitaufwendigen Abhärtungsprozeß unterworfen, nicht an die Freiluftverhältnisse adaptiert zu werden.

Die "abhärtenden" Faktoren können in der Gewebekultur offensichtlich nicht hergestellt werden. Möglich schien es jedoch, den einen oder anderen Faktor zu modellieren.

Die Wasseraufnahme der Pflanzen beruht auf dem osmotischen Druck, genauer: auf dem Unterschied zwischen dem osmotischen Druck des Bodens und dem in der Wurzel der Pflanze herrschenden osmotischen Druck. Der isoosmotische Druck der Pflanzen entspricht unter natürlichen Verhältnissen etwa dem osmotischen Druck einer 3 M Zuckerlösung, unter in vitro Bedingungen ist er etwas niedriger, etwa 0,25 M. Wenn Trockenheit herrscht, ist der osmotische Druck des Bodens größer als der osmotische Druck in den Zellen der Pflanzenwurzel, deswegen entzieht der Boden den Wurzeln Wasser, die Pflanzen dursten. Wenn der osmotische Druck des Bodens größer ist als der isoosmotische Druck der Pflanzen, kann der Wasserentzug derart stark sein, daß die Verbindung der einzelnen Zellen miteinander infolge der innerhalb der Zellen erfolgenden Protoplastbildung ganz aufhört.

Die Erfindung beruht auf der Erkenntnis, daß in den Anzuchtgefäßen, in denen im übrigen eine relative Luftfeuchte von 100 % herrscht, für die Pflanze ein Zustand des Wassermangels hervorgerufen werden kann, wenn man dem Nährboden wenigstens einen nicht-toxischen, zur Einstellung des osmotischen Druckes geeigneten Zusatz in einer Konzentration zusetzt, die den osmotischen Druck einer 0,2-0,4 M Zuckerlösung erzeugt, und/oder wenn man die Temperatur anhaltend auf 2-10 °C absenkt. Die Verminderung der Temperatur ruft ebenfalls einen Wassermangel in der Pflanze hervor, weil die Membranprozesse der Wasseraufnahme von der Temperatur abhängen. Durch Variieren der beiden erwähnten Faktoren - osmotischer Druck und Temperatur - kann die abhärtende Wirkung der natürlichen Freilandbedingungen bereits im Abschnitt der Gewebekultur simuliert werden, und dadurch wird die Pflanze zum unmittelbar erfolgenden Auspflanzen geeignet. För die Pflanze ist es gleichgültig, ob kein Wasser da ist, oder ob welches da ist, sie es aber nicht aufnehmen kann: auf den erhöhten osmotischen Druck des Nährbodens beziehungsweise auf die verminderte Temperatur reagiert die Pflanze in überraschender Weise mit intensiver Wurzelbildung und einer Umwandlung ihres Habitus.

Gegenstand der Erfindung ist demnach ein Verfahren zur Adaptierung, Akklimatisierung von durch Gewebekultur vermehrten Pflanzen für unmittelbares Auspflanzen ins Freiland. Für das Verfahren ist kennzeichnend, daß man in bekannter Weise durch Gewebekultur vermehrte Pflanzen
a) in einem oder in mehreren Schritten auf Nährböden weiterzüchtet, deren osmotischer Druck 0,2-0,4 M Saccharosegehalt entspricht, wobei im Falle einer mehrstufigen Anzucht der osmotische Druck des Nährbodens innerhalb des angegebenen Bereiches von Stufe zu Stufe auf einen höheren Wert eingestellt wird, und/oder
b) wenigstens 30 Tage lang bei 2-10 °C lagert.

In überraschender Weise sind die auf die beschriebene Weise gezogenen Pflanzen geeignet, unmittelbar, ohne jede Abhärtung sofort ins Freiland ausgepflanzt oder bei niedriger Temperatur (5-10 °C) 3-4 Monate gelagert zu werden.

An die zur Einstellung des osmotischen Druckes verwendeten Zusätze werden nur zwei Anforderungen gestellt. Die eine ist, daß die Substanz den osmotischen Druck überhaupt beeinflußt. Zahlreiche natürliche Oligo- und Polimere, z.B. Agar, Gelatine, haben nur einen verschwindend geringen osmotischen Druck (d'Ans, Lax: Taschenbuch für Chemiker und Physiker, 2. Auflage, S. 909, Springer-Verlag Berlin-Göttingen-Heidelberg)

Wie bekannt, hängt der osmotische Druck nicht von der Natur des verwendeten Stoffes ab, sondern nur von der Anzahl der im Medium vorhandenen Teilchen. 34,2 g Saccharose (Rohrzucker) in einem Liter Wasser (0,1 Mol) üben zum Beispiel den gleichen osmotischen Druck aus wie 3,73 g/l KCl, weil bei letzterem nicht nur die kleinere Molmasse berücksichtigt werden muß, sondern auch die Tatsache, daß das Salz dissoziiert und dadurch die Chlorionen und die Kaliumionen getrennt als Teilchen zum osmotischen Druck beitragen. Diese Tatsachen sind dem Fachmann bekannt, und die erforderliche Menge des verwendeten Stoffes kann leicht berechnet werden. Die zweite Forderung ist, daß der Zusatzstoff nicht phytotoxisch sein darf. Das schließt zum Beispiel die Natriumsalze aus.

Unter den prinzipiell verwendeten Stoffen sind diejenigen bevorzugt, die in der Technik der Gewebekultur ohnehin angewendet werden, zum Beispiel Saccharose, sonstige Zucker, die Zuckeralkohole, die für das Nährmedium verwendeten Nährsalze usw.

Da die auf dem osmotischen Druck beruhende Wasseraufnahme der Pflanzen ein ziemlich universelles Prinzip ist, kann das erfindungsgemäße Verfahren für verholzende pflanzen und für keinen verholzenden Stiel ausbildende Pflanzen, für einjährige und mehrjährige Pflanzen gleichermaßen angewendet werden, vorausgesetzt, die betreffende Pflanze ist überhaupt für die Vermehrung durch Gewebekultur geeignet. Im folgenden werden einige Pflanzen aufgezählt, an denen das erfindungsgemäße Verfahren bereits mit Erfolg angewendet wurde

Zierbäume und Ziersträucher, zum Beispiel Weiden und Rhododentron;
Gemüsearten, wie Spargel, Tomaten, Brokkoli, Chikoree, Kürbis, Melonen, Kohlrabi, Gurken, Kartoffeln, Rüben;
Topfplanzen, wie die Flamingoblume, der Gummibaum, das Usambaraveilchen, Philodendron, Bromelien, Dieffenbachia, Begonien, Cyklamen, die Drachenlilie und Geranien;
Zwiebelpflanzen, wie Gladiolen, Freesien, Hyacinten, Schwertlilien und andere Lilien;
Schnittblumen, wie Astern, Rosen, Chrysanthemen, Orchideen, Glockenblumen, Nelken und Gerbera.

Die erfindungsgemäß angezogenen Pflanzen können direkt aufs Feld, in den Garten oder in die Baumschule ausgepflanzt werden. Ihre Uberlebensrate ist höher als die der dem üblichen Adaptionsverfahren unterzogenen Pflanzen. Da das Auspflanzen direkt auf den Abschnitt der Gewebekultor folgt, wurde das Verfahren als Tissue-Culture-Direct-Verfahren, und die hergestellten Pflanzen wurden als TCD-Pflanzen bezeichnet.

Das Verfahren wird im folgenden an Hand von Beispielen näher erläutert, ist jedoch nicht auf diese Beispiele beschränkt.

### Beispiel 1

### Limonium latifolium Zweistufiges Verfahren

Limonium latifolium (eine mehrjährige Zierpflanze) wurde auf einem herkömmlichen Nährboden der folgenden Zusammensetzung vermehrt:

| | | | |
|---|---|---|---|
| NH₄NO₃ | 1600 mg | CoCl₂ . 6 H₂O | 0,025 mg |
| KNO₃ | 1900 mg | Mesoinosit | 100 mg |
| CaCl₂ . 2H₂O | 440 mg | Vitamin B₁ | 0,1 mg |
| MgSO₄ . 7H₂O | 370 mg | Vitamin B₆ | 0,5 mg |
| Na₂EDTA+FeSO₄.7H₂O | 25 mg | Nikotinsäure | 0,5 mg |
| H₃BO₃ | 6,2 mg | Glycin | 2,0 mg |
| MnSO₄.4H₂O | 22,3 mg | Kinetin | 2,0 mg |
| ZnSO₄.4H₂O | 8,6 mg | α-Naphthylessigs. | 0,02 mg |
| KJ | 0,83 mg | Saccharose | 30 000 mg |
| Na₂MoO₄.2H₂O | 0,25 mg | Agar | 11 000 mg |
| CuSO₄.5H₂O | 0,025 mg | pH 5,7 | |

Diese Stoffe sind in einem Liter Nährboden enthalten, d.h. der Nährboden enthält 3 % Saccharose und 1,1 % Agar. Es sei bemerkt, daß der Saccharosegehalt dieses herkömmlichen Nährbodens weit unter der zur Einstellung des osmotischen Druckes auf den gewünschten Wert erforderlichen Menge liegt. In diesem Nährboden dient die Saccharose als Energiequelle für die unter den Bedingungen der Gewebekultur kaum assimilierenden Pflanzen.

Nachdem eine genügende Vermehrung erzielt wurde (etwa 30 000 Stück), wurden die Pflanzen auf einen Bewurzelungsnährboden der folgenden Zusammensetzung umgesetzt.

| | | | |
|---|---|---|---|
| NH₄NO₃ | 1600 mg | CoCl₂ . 6 H₂O | 0,025 mg |
| KNO₃ | 1900 mg | Mesoinosit | 100 mg |
| CaCl₂ . 2H₂O | 440 mg | Vitamin B₁ | 0,5 mg |
| MgSO₄ . 7H₂O | 370 mg | Vitamin B₆ | 1,0 mg |
| Na₂EDTA+FeSO₄.7H₂O | 25 mg | Nikotinsäure | 5,0 mg |
| H₃BO₃ | 6,2 mg | Pantothensäure | 2,5 mg |
| MnSO₄.4H₂O | 22,3 mg | α-Naphthylessigs. | 0,02 mg |
| ZnSO₄.4H₂O | 8,6 mg | Saccharose | 70 000 mg |
| KJ | 0,83 mg | Agar | 11 000 mg |
| Na₂MoO₄.2H₂O | 0,25 mg | pH 5,7 | |

Dabei kamen jeweils 10 Pflanzen in ein Glas von 400 ml Volumen. Der Zuckergehalt und der osmotische Druck des Bewurzelungsnährbodens sind mehr als doppelt so hoch wie die entsprechenden Werte des Vermehrungsnährbodens. Der Bewurzelungsnährboden hat einen osmotischen Druck, der dem osmotischen Druck von 0,21 M Saccharose entspricht. Auf diesem Nährboden begann die Umwandlung der Pflanzen. Als die Pflanzen eine Höhe von 5-6 cm erreicht hatten und die Wurzelbildung begann, wurden sie auf einen dritten Nährboden umgepflanzt, der sich von dem zweiten Nährboden, dem Bewurzelungsnährboden nur darin unterscheidet, daß er 110 g/l (0,32 Mol) Saccharose und 250 mg/l Aktivkohle enthält. Die Aktivkohle hatte die Aufgabe, die bereits gebildeten Wurzeln vor dem Licht zu schützen. Je 25 ml dieses Nährbodens wurden in 6 cm hohe, leicht kegelförmige durchsichtige Kunststoffbecher gefüllt, nach dem Erstarren des Nährbodens wurde in jeden Becher eine Pflanze eingesetzt und der Becher dann mit einem sterilen Deckel verschlossen. Die Becher wurden bei 22-25 °C und Tagesbeleuchtung gehalten, bis das Wurzelwerk den Nährboden völlig durchwachsen hatte. Damit war die TCD-Pflanze fertig, sie konnte ohne Adaptierung ins Freiland ausgesetzt beziehungsweise im Kühlraum bei 5-10 °C 3-4 Monate lang gelagert werden.

### Beispiel 2

### Herstellung von Spathyphylum-TCD-Pflanzen

Von den in herkömmlicher Weise durch Gewebekultur vermehrten Spathyphylum-Pflanzen (für Blumentöpfe geeignete Zierpflanze) wurden sog. Mikrostecklinge, d.h. Triebe ohne Wurzel, geschnitten. Diese wurden in 6 cm hohe Kunststoffbecher gesetzt, die bis zu 2-3 cm Höhe mit dem Bewurzelungsnährboden gemäß Beispiel 1 gefüllt waren. In jeden Becher wurden drei Mikrostecklinge beziehungsweise ein sich dreifach verzweigender Mikrosteckling eingesetzt. Die Becher wurden bei 22 °C und Tagesbeleuchtung gehalten. Die Pflanzen erreichten in etwa 6 Wochen die TDC-Qualität. Sie konnten ohne im Gewächshaus erfolgende Adaptierung sofort in Töpfe gepflanzt und im Handel angeboten werden.

### Beispiel 3

### Gerbera jamesoni als TCD-Pflanze

Gerbera jamesoni wurde in gewohnter Weise durch Gewebekultur vermehrt und dann bewurzelt. Als die Wurzeln eine Länge von 1-1,5 cm erreicht hatten, wurden die Pflanzen in Kunststoffbecher umgesetzt, die bis zu 2-3 cm Höhe mit Nährboden gefüllt waren. Der Nährboden entsprach dem Bewurzelungsnährboden gemäß Beispiel 1, enthielt jedoch 130 g/l Saccharose und 500 mg/l Aktivkohle. Nach 6 Wochen konnten die Pflanzen ausgepflanzt werden.

### Beispiel 4

### Herstellung von TCD-Gurkenpflanzen in zwei Stufen

Die Clone der in bekannter Weise durch Gewebekultur vermehrten F1-Hybriden von Gurken wurden auf Bewurzelungsnährboden gelegt. Dessen Zusammensetzung entsprach der im Beispiel 1 angegebenen, jedoch enthielt er 50 g/l Saccharose und 0,01 mg/l Naphthylessigsäure. Auf diesem Nährboden wurden die Pflanzen gezogen, bis sie 4-5 Blätter hatten, dann wurden die bewurzelten Pflanzen auf einen Nährboden umgesetzt, der 50 g/l Glucose und 0,01 mg/l Naphthylessigsäure enthielt (der osmotische Druck von 50 g/l Glucose entspricht dem osmotischen Druck von 100 g Saccharose). Auf diesem Nährboden werden die Pflanzen noch wenigstens 2 Wochen lang kultiviert und dann ausgepflanzt oder bei 5-10 °C gelagert.

### Beispiel 5

### Herstellung von TCD-Spargelpflanzen

### a) Vorbereitung des Vermehrungsmaterials

Die in bekannter Weise bewurzelten Pflanzen wurden steril auf den verfestigten Nährboden aufgebracht. Die Zusammensetzung des Nährbodens entsprach der des ersten Nährbodens in Beispiel 1, jedoch waren keine pflanzlichen Hormone enthalten. Die mit 15-20 Pflanzen besetzten Glaskolben wurden bei 17 °C und Kurztagbeleuchtung 21 Tage lang inkubiert. Die Inkubation war beendet, wenn neben dem frischen Trieb eine Knospe von 1-2 mm Länge erschien. Die im Ruhezustand befindlichen Knospen wurden zusammen mit einem Stück Wurzel abgetrennt, oder die Wurzeln wurden geteilt, und die Stücke wurden in frische Kolben gesetzt, bis etwa 10 000 Versuchspflanzen bereitstanden.

### b) Herstellung von TCD-Pflanzen durch Kühlen

Ein Teil der hergestellten Pflanzen wurde in die Kühlkammer gebracht und dort bei 5 °C gelagert. Alle 10 Tage wurden einige Kolben entnommen, die in ihnen enthaltenen Pflanzen wurden bei einer Bodentemperatur von 25 °C (für Spargel optimal) ausgepflanzt. Vor dem Auspflanzen wurden die alten Triebe in 0,5 cm Höhe zurückgeschnitten. Es wurde dafür gesorgt, daß der Boden über längere Zeit hinweg unkrautfrei blieb. Vor dem Pflanzen wurde der Boden bewässert, dann wurde eine 12 cm tiefe Rinne gezogen, und in diese wurde ein das Wasser zurückhaltender Stoff (quellbare Acryl-polymere, Torf usw.) eingearbeitet. Auf diese Schicht wurden die Pflanzen aufgesetzt und dann mit einer 8-10 cm dicken Erdschicht bedeckt. Der Boden wurde mit einer leichten Walze verdichtet. Die in Abständen von 10 Tagen gesetzten Pflanzen wurden beobachtet. Das Auflaufen als Funktion der Kühldauer ist in der folgenden Tabelle dargestellt.

| Lagerzeit bei 5 °C Tage | Aufgelaufene Pflanzen, % | Auflaufzeit Tage |
|---|---|---|
| 0 | 11 | 40 |
| 10 | 50 | 25 |
| 30 | 80 | 15 |
| 40 | 96 | 10 |
| 50 | 100 | 10 |

Es ist ersichtlich, daß die erfindungsgemäß behandelten Pflanzen sicher auflaufen, und zwar schneller als die unbehandelten, von denen nur einige aufliefen, und das erst nach 40 Tagen.

### c) Herstellung von TCD-Pflanzen durch Veränderung des osmotischen Druckes

Der andere Teil der gemäß Punkt a) vorbereiteten Pflanzen wurde auf einen Nährboden gepflanzt, der dem Bewurzelungsnährboden gemäß Beispiel 1 entsprach, aber pro Liter 0,3 Mol Saccharose enthielt. Die Pflanzen wurden bei kontinuierlicher Beleuchtung und 25 °C auf diesem Nährboden 30 Tage lang kultiviert, dann aus den Kolben herausgenommen, und die an ihnen befindlichen alten Triebe wurden wie unter b) beschrieben zurückgeschnitten. Die Pflanzen wurden auf die beschriebene Weise in 25 °C warmen Boden gesetzt. Die Pflanzen verhielten sich wie eine normale Spargelpflanzung.

## Patentansprüche

1. Verfahren zur Adaptierung von durch Gewebekultur vermehrten Pflanzen für unmittelbares Auspflanzen ins Freiland, dadurch gekennzeichnet, daß man die in bekannter Weise durch Gewebekultur vermehrten Pflanzen
a) in einem oder in mehreren Schritten auf Nährböden weiterzüchtet, deren osmotischer Druck 0,2-0,4 M Saccharosegehalt entspricht, wobei im Falle einer mehrstufigen Anzucht der osmotische Druck des Nährbodens innerhalb des angegebenen Bereiches von Stufe zu Stufe auf einen höheren Wert eingestellt wird, und/oder
b) wenigstens 30 Tage lang bei 2-10 °C lagert, und die auf diese Weise erhaltenen Pflanzen unmittelbar ins Freiland auspflanzt oder für diesen Zweck vermarktet.

2. Verfahren nach Anspruch 1a), dadurch gekennzeichnet, daß man zur Einstellung des osmotischen Druckes Saccharose, Glucose, pflanzliche Nährsalze oder beliebige Gemische der aufgeführten Stoffe verwendet.

3. Verfahren nach Anspruch 1a), dadurch gekennzeichnet, daß man die Pflanzen zusammen mit dem Nährboden des letzten Schrittes unter sterilen Bedingungen einzeln in durchsichtige Kunststoffbecher pflanzt und in diesen weiter kultiviert.

4. Verfahren nach Anspruch 1b), dadurch gekennzeichnet, daß man die Pflanzen bei 5 °C 30-50 Tage lang lagert.
